# EUROPEAN PATENT APPLICATION

(11) **EP 2 491 933 A1**
(43) Date of publication of application: **29.08.2012**
(21) Application number: 10825040.8
(22) Date of filing: 22.10.2010
(51) Int. Cl.: A61K 31/7042, A61K 9/16, A61K 9/20, A61K 31/401, A61K 47/36, A61K 47/38

(54) **PHARMACEUTICAL COMPOSITION FOR ORAL ADMINISTRATION**

(30) Priority: 23.10.2009 US 254443
(71) Applicant: Astellas Pharma Inc., Tokyo 103-8411 (JP); Kotobuki Pharmaceutical Co., Ltd., Hanishina-gun, Nagano 389-0697 (JP)
(72) Inventor: SAKAURA, Keisuke, Tokyo 103-8411 (JP); KATAKAWA, Yoshifumi, Tokyo 103-8411 (JP); TAMURA, Tetsuya, Tokyo 103-8411 (JP); SAKO, Kazuhiro, Tokyo 103-8411 (JP)
(74) Representative: HOFFMANN EITLE
(86) International application number: PCT/JP2010/068660
(87) International publication number: WO 2011/049191

(57) **Abstract**

The present invention provides a solid pharmaceutical composition comprising a cocrystal of (1S)-1,5-anhydro-1-[3-(1-benzothien-2-ylmethyl)-4-fluorophenyl]-D-glucitol and L-proline, in combination with crystalline cellulose, and also provides a method for producing the composition.

## Description

### TECHNICAL FIELD

The present invention relates to a solid pharmaceutical composition, which comprises a cocrystal of (1S)-1,5-anhydro-1-[3-(1-benzothien-2-ylmethyl)-4-fluorophenyl]-D-glucitol and L-proline and which maintains good dissolution properties and dissolution stability of (1S)-1,5-anhydro-1-[3-(1-benzothien-2-ylmethyl)-4-fluorophenyl]-D-glucitol.

The present invention also relates to a method for producing a solid pharmaceutical composition, which comprises a cocrystal of (1S)-1,5-anhydro-1-[3-(1-benzothien-2-ylmethyl)-4-fluorophenyl]-D-glucitol and L-proline and which maintains good dissolution properties and dissolution stability of (1S)-1,5-anhydro-1-[3-(1-benzothien-2-ylmethyl)-4-fluorophenyl]-D-glucitol.

The present invention further relates to the use of crystalline cellulose for the manufacture of a solid pharmaceutical composition, which comprises a cocrystal of (1S)-1,5-anhydro-1-[3-(1-benzothien-2-ylmethyl)-4-fluorophenyl]-D-glucitol and L-proline and which maintains good dissolution properties and dissolution stability of (1S)-1,5-anhydro-1-[3-(1-benzothien-2-ylmethyl)-4-fluorophenyl]-D-glucitol.

### BACKGROUND ART

(1S)-1,5-Anhydro-1-[3-(1-benzothien-2-ylmethyl)-4-fluorophenyl]-D-glucitol (hereinafter referred to as "C-glycoside derivative A" or "known compound A") is a Na⁺-glucose cotransporter inhibitor designed and developed by Astellas Pharma Inc. and is reported as a compound useful in treating and preventing, for example, insulin-dependent diabetes (type I diabetes), non-insulin-dependent diabetes (type II diabetes), as well as insulin resistance diseases and obesity (Patent Literature 1: see Example 138).

Moreover, there are disclosed inventions relating to a cocrystal of known compound A and L-proline, i.e., an invention of a cocrystal with L-proline, which cocrystal has a consistent quality and is excellent in storage stability as a crystal of a drug substance used for medicament production, and an invention of a pharmaceutical composition comprising this cocrystal as an active ingredient, which composition is particularly useful as a therapeutic agent for diabetes (Patent Literature 2).

### CITATION LIST

### PATENT LITERATURE

PTL 1: WO2004/080990
PTL 2: WO2007/114475

### SUMMARY OF INVENTION

### TECHNICAL PROBLEM

The crystal of known compound A disclosed in Patent Literature 1 forms a clathrate hydrate and reversibly changes from an anhydride form to a non-stoichiometric hydrate on a hygrothermal condition. Thus, known compound A has not been able to retain a consistent quality as a drug substance for use in medicaments. For this reason, known compound A is provided in the form of a cocrystal with L-proline, which cocrystal has a consistent quality and is also excellent in storage stability as a crystal of a drug substance for use in medicaments.

However, when capsule formulations containing a cocrystal of known compound A and L-proline were produced, the cocrystal of known compound A and L-proline was found to be problematic in that the cocrystal was subject to poor disintegration due to its strong aggregation tendency, which caused a reduction in drug dissolution speed. Thus, there is a concern that poor disintegration of the formulations and reduced drug dissolution speed will give rise to further problems, such as reduced bioavailability (BA) and lack of any pharmacologically sufficient therapeutic effect.

Thus, an object of the present invention is to provide a pharmaceutical composition having good dissolution properties, which contains known compound A in the form of a cocrystal with L-proline.

Another object of the present invention is to provide a method for producing a pharmaceutical composition having good dissolution properties, which contains known compound A in the form of a cocrystal with L-proline.

Yet another object of the present invention is to provide the use of crystalline cellulose for the manufacture of a solid pharmaceutical composition, which contains a cocrystal of known compound A and L-proline and which maintains good dissolution properties and dissolution stability of known compound A.

### SOLUTION TO PROBLEM

The inventors of the present invention prepared a granulated product containing a cocrystal of known compound A and L-proline (1:1) by wet granulation process using a stirring granulator known per se, and then produced tablets from this granulated product. As a result, the inventors found that these tablets showed good drug dissolution properties immediately after production, but were subject to problems such as a change in their disintegration properties and a time-induced reduction in their dissolution properties.

The inventors of the present invention have conducted studies focusing on the state of a drug during production of formulations, and have found the following: known compound A is converted into a free form upon release of L-proline from the cocrystal structure by the action of water used during production of formulations; drug dissolution properties are temporarily improved based on the properties of the free form of known compound A; and as time passes, the free form of known compound A associates again with L-proline remaining in the composition through hydrogen bonding or other interactions to form an aggregate.

The inventors of the present invention conducted extensive and intensive studies of solid pharmaceutical compositions having good dissolution properties while maintaining the cocrystal structure between known compound A and L-proline. As a result of studies by using D-mannitol, anhydrous dibasic calcium phosphate, lactose, crystalline cellulose and the like as an excipient, the inventors found that a pharmaceutical composition containing crystalline cellulose has good dissolution properties while maintaining the cocrystal structure between known compound A and L-proline, and completed the present invention.

Namely, the present invention relates to
[1] A solid pharmaceutical composition comprising a cocrystal of (1S)-1,5-anhydro-1-[3-(1-benzothien-2-ylmethyl)-4-fluorophenyl]-D-glucitol and L-proline, in combination with crystalline cellulose;
[2] The solid pharmaceutical composition according to [1], wherein the content of crystalline cellulose in the pharmaceutical composition is 5% by weight or more to 90% by weight or less;
[3] The solid pharmaceutical composition according to [1] or [2], which further comprises a disintegrant;
[4] The solid pharmaceutical composition according to [3], wherein the disintegrant is one or more members selected from the group consisting of sodium starch glycolate, and hydroxypropylcellulose whose hydroxypropoxyl group content is 5% by weight or more to less than 16% by weight;
[5] The solid pharmaceutical composition according to [3] or [4], wherein the content of the disintegrant in the pharmaceutical composition is 5% by weight or more to 90% by weight or less;
[6] The solid pharmaceutical composition according to any one of [1] to [5], wherein 65% or more of (1S)-1,5-anhydro-1-[3-(1-benzothien-2-ylmethyl)-4-fluorophenyl]-D-glucitol is dissolved in 30 minutes, as analyzed by the dissolution test described in the 15th revised Japanese Pharmacopoeia;
[7] A method for producing a solid pharmaceutical composition, which comprises the steps of:
   (1) mixing a cocrystal of (1S)-1,5-anhydro-1-[3-(1-benzothien-2-ylmethyl)-4-fluorophenyl]-D-glucitol and L-proline with crystalline cellulose; and
   (2) granulating the resulting mixture by wet granulation while maintaining the cocrystal structure between (1S)-1,5-anhydro-1-[3-(1-benzothien-2-ylmethyl)-4-fluorophenyl]-D-glucitol and L-proline;
[8] The method according to [7], which further comprises the step of (3) subjecting the granulated product to compression molding;
[9] The method according to [7] or [8], wherein the content of crystalline cellulose in the pharmaceutical composition is 5% by weight or more to 90% by weight or less;
[10] The method according to [7], which further comprises adding a disintegrant during step (1), between steps (1) and (2), during step (2), or after step (2);
[11] The method according to [10], wherein the disintegrant is one or more members selected from the group consisting of sodium starch glycolate, and hydroxypropylcellulose whose hydroxypropoxyl group content is 5% by weight or more to less than 16% by weight;
[12] The method according to [10] or [11], wherein the content of the disintegrant in the pharmaceutical composition is 5% by weight or more to 90% by weight or less;
[13] The method according to any one of [7] to [12], which is intended to produce a solid pharmaceutical composition that allows 65% or more of (1S)-1,5-anhydro-1-[3-(1-benzothien-2-ylmethyl)-4-fluorophenyl]-D-glucitol to be dissolved in 30 minutes, as analyzed by the dissolution test described in the 15th revised Japanese Pharmacopoeia;
[14] The method according to [7], wherein the wet granulation is performed such that the solid pharmaceutical composition has a maximum moisture content value of 5% to 30% by weight during granulation;
[15] Use of crystalline cellulose for the manufacture of a solid pharmaceutical composition comprising a cocrystal of (1S)-1,5-anhydro-1-[3-(1-benzothien-2-ylmethyl)-4-fluorophenyl]-D-glucitol and L-proline.

### ADVANTAGEOUS EFFECTS OF INVENTION

The present invention is characterized, for example, in that (1) pharmaceutical formulations containing a cocrystal of known compound A and L-proline show good dissolution properties, (2) it is possible to provide stable pharmaceutical formulations whose dissolution speed remains unchanged over time, and (3) the pharmaceutical formulations show improved bioavailability (BA) and exert a pharmacologically sufficient therapeutic effect due to their good dissolution properties.

### BRIEF DESCRIPTION OF DRAWINGS

Figure 1 shows the dissolution profile of the pharmaceutical compositions obtained in Example 1.
Figure 2 shows the dissolution profile of the pharmaceutical compositions obtained in Comparative Example 1.
Figure 3 shows the dissolution profile of the pharmaceutical compositions obtained in Comparative Example 2.
Figure 4 shows the dissolution profile of the pharmaceutical compositions obtained in Examples 3, 4, 5, 6, and 9.

### DESCRIPTION OF EMBODIMENTS

The solid pharmaceutical composition of the present invention will be further described in more detail below.

As used herein, the term "cocrystal of known compound A and L-proline" is intended to mean a uniform cocrystal formed between known compound A and L-proline at a molar ratio of 1:1. The cocrystal structure is identified from the results of differential scanning calorimeter analysis (DSC analysis) and powder X-ray diffractometry, etc. For example, in the case of powder X-ray diffractometry, a distinction can be made based on diffraction angles (2θ (°)) and relative intensity in the measured spectra (Table 1, Table 2). It should be noted that crystal lattice spacings and overall pattern are important for identification of crystal in powder X-ray diffractometry due to the characteristics of the data, and the relative intensity should not be strictly interpreted because it may more or less vary depending on the direction of crystal growth, particle sizes and measurement conditions. Moreover, if the peaks unique to the structure of known compound A are as small as negligible in X-ray diffractometry, such a case is defined to mean a cocrystal of known compound A and L-proline.

[Table 1]

**Table 1: Diffraction angle (2θ (°)) and relative intensity of known compound A**

| Diffraction angle | Relative intensity | Diffraction angle | Relative intensity |
|---|---|---|---|
| 9.80 | Medium | 18.8 | Strong |
| 11.9 | Medium | 20.1 | Strong |
| 15.4 | Medium | 23.9 | Strong |

[Table 2]

**Table 2: Diffraction angle (2θ (°)) and relative intensity of cocrystal of known compound A and L-proline**

| Diffraction angle | Relative intensity | Diffraction angle | Relative intensity |
|---|---|---|---|
| 4.14 | Medium | 18.8 | Strong |
| 8.98 | Medium | 20.1 | Strong |
| 12.4 | Medium | 23.9 | Strong |
| 16.5 | Medium | 21.5 | Medium |

Powder X-ray diffractometry was conducted under the following conditions.
(1) Standard measurement: "MAC Science MXP18TAHF22" was used in the measurement under the following conditions: X-ray tube: Cu, tube current: 200 mA, tube voltage: 40 kV, sampling width: 0.020°, scanning speed: 3°/min, wavelength: 1.54056 Å, measured range of diffraction angle (2θ): 3° to 40°.
(2) Humidity-controlled measurement: "MAC Science MXP18TAHF22 with a multifunctional humidity and temperature converter (VAISALA MHP235)" was used in the measurement under the following conditions: X-ray tube: Cu, tube current: 350 mA, tube voltage: 50 kV, sampling width: 0.020°, scanning speed: 3°/min, wavelength: 1.54056 Å, measured range of diffraction angle (2θ): 5° to 40°.

As used herein, the term "good dissolution properties" is intended to mean having dissolution properties equal or equivalent to those of standard formulations. For example, this term is used herein in a definition stating that the dissolution rate after 30 minutes is 65% or more, or alternatively, 75% or more, or alternatively, 80% or more, as analyzed by the dissolution test described in the 15th revised Japanese Pharmacopoeia.

As used herein, the term "dissolution stability" is intended to mean that in drug dissolution from a pharmaceutical composition, there is little time-induced change in the drug dissolution rate as compared to before storage. For example, this term is used herein in a definition stating that there is little time-induced change in the drug dissolution rate as compared to before storage, as analyzed by the dissolution test described in the 15th revised Japanese Pharmacopoeia. Alternatively, use is made of the term in a definition stating that when tested after storage at 40°C and 75% for 6 months, the dissolution rate measured at 30 minutes after the start of the dissolution test was within ± 15% as compared to before storage.

Known compound A used in the present invention is represented by the following formula (I):

[Formula 1] 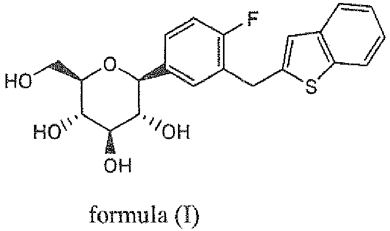 and has the chemical name of (1S)-1,5-anhydro-1-[3-(1-benzothien-2-ylmethyl)-4-fluorophenyl]-D-glucitol (hereinafter also referred to as "C-glycoside derivative A" or simply "known compound A"). Known compound A forms a cocrystal structure with L-proline, as represented by the following formula (II).

[Formula 2]

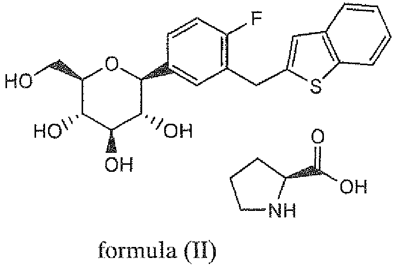

This cocrystal has an endothermic peak at 201°C to 213°C in DSC analysis, and/or has peaks around 2θ (°) = 4.44, 8.98, 12.4, 16.5, 17.5, 18.7, 20.5 and 21.5 in powder X-ray diffractometry.

Known compound A and a cocrystal of known compound A and L-proline can be distinguished from each other based on diffraction angles (2θ (°)) and relative intensity in their powder X-ray diffraction spectra.

Although the clinical dosage (therapeutically effective dosage) of a cocrystal of known compound A for humans is appropriately determined taking into account the symptoms, weight, age, sex and the like of a patient to be administered, the daily dosage for adults is usually 0.1 to 500 mg by the oral route, given as a single dose or in divided doses. Since the dosage will vary according to various conditions, a dosage smaller than the above range is sufficient in some cases.

Crystalline cellulose to be used in the present invention is purified, partially depolymerized cellulose prepared by acid treatment of α-cellulose obtained as a pulp from fibrous plants (15th revised Japanese Pharmacopoeia). Moreover, crystalline cellulose can be used without any limitations on its bulk density and average polymerization degree, etc., as long as it is pharmaceutically acceptable and capable of maintaining good dissolution properties and dissolution stability of known compound A. Specific examples include Ceolus PH101, Ceolus PH102, Ceolus PH101D, Ceolus KG802, Ceolus UF711, Ceolus UF702, Ceolus KG1000, Ceolus PH301, Ceolus PH301D, Ceolus PH301Z, Ceolus PH302, Ceolus PH F20JP (all available from Asahi Kasei Corporation, Japan), Avicel PH101, Avicel PH112, Avicel PH113, Avicel PH200, Avicel PH301, Avicel PH302, Avicel HFE-102, Avicel (all available from FMC BioPolymer), Celex 101 (International Specialty Products), Emcocel 90M (J.Rettenmaier & Sohne), Vivacel 12 (J.Rettenmaier & Sohne), Celphere (San-Ei Gen F.F.I., Inc., Japan) and the like.

Crystalline cellulose may be of any shape, including granular or needle-like shape. Crystalline cellulose of needle-like shape may further be ground before use. It is also possible to use crystalline cellulose that is commercially available as a mixture associated with another additive (e.g., carrageenan, sodium carboxymethylcellulose, guar gum). In a case where crystalline cellulose is of granular shape, its average particle size is preferably 20 to 200 µm, as analyzed by the second method (analytical sieving) of powder particle size determination described in the Japanese Pharmacopoeia. Crystalline celluloses of different grades, shapes, average particle sizes or the like may be used alone or in combination as appropriate for this purpose.

In general, the content of crystalline cellulose is not limited in any way as long as it ensures good dissolution properties of known compound A. For example, it is 5% to 90% by weight, or alternatively, 20% to 70% by weight, based on the pharmaceutical compositions of the present invention. Likewise, it is 20% to 1500% by weight, or alternatively, 50% to 1100% by weight or 40% to 350% by weight, relative to the amount of the cocrystal of known compound A and L-proline.

In general, any type of disintegrant may be used in the present invention as long as known compound A shows good dissolution properties. Examples include low-substituted hydroxypropylcellulose, sodium starch glycolate, corn starch, potato starch, carmellose calcium, carmellose sodium, partially pregelatinized starch, crospovidone, croscarmellose sodium and the like. In other embodiments, low-substituted hydroxypropylcellulose and sodium starch glycolate can be presented. These disintegrants may be used alone or in combination as appropriate for this purpose.

In general, the content of disintegrant is not limited in any way as long as it ensures good dissolution properties of known compound A. For example, it is 5% to 90% by weight, or alternatively, 5% to 70% by weight or 5% to 55% by weight, based on the pharmaceutical composition of the present invention. Likewise, it is 10% to 1500% by weight, or alternatively, 10% to 1100% by weight or 25% to 300% by weight, relative to the amount of the cocrystal of known compound A and L-proline.

Low-substituted hydroxypropylcellulose is not limited in any way as long as it is pharmaceutically acceptable. An example is hydroxypropylcellulose whose hydroxypropoxyl group content is 5% by weight or more to less than 16% by weight. Specific examples include L-HPCs (LH-11, LH-21, LH-22, LH-B1, LH-31, LH-32, LH-B1; all available from Shin-Etsu Chemical Co., Ltd., Japan) and the like. Low-substituted hydroxypropylcellulose may be of any shape, including granular or fiber-like shape. Low-substituted hydroxypropylcellulose of fiber-like shape may further be ground before use. In a case where low-substituted hydroxypropylcellulose is of granular shape, its average particle size is preferably 10 to 100 µm, as analyzed by the second method (analytical sieving) of powder particle size determination described in the Japanese Pharmacopoeia. Such low-substituted hydroxypropylcelluloses may be used alone or in combination as appropriate for this purpose.

The content of low-substituted hydroxypropylcellulose is 5% to 90% by weight, or alternatively, 5% to 70% by weight or 5% to 55% by weight, based on the pharmaceutical composition of the present invention. Likewise, it is 10% to 1500% by weight, or alternatively, 10% to 1100% by weight or 25% to 300% by weight, relative to the amount of the cocrystal of known compound A and L-proline.

Sodium starch glycolate is not limited in any way as long as it is pharmaceutically acceptable. Examples include Primojel (DMV), Explotab (Kimura Sangyo Co., Ltd., Japan) and the like. Sodium starch glycolate may be of any shape, including granular, needle-like, egg-like, or spherical shape. Sodium starch glycolate of needle-like shape may further be ground before use. In a case where sodium starch glycolate is of granular shape, for example, its average particle size is preferably 10 to 100 µm, as analyzed by the second method (analytical sieving) of powder particle size determination described in the Japanese Pharmacopoeia. Sodium starch glycolates of different grades or the like may be used alone or in combination as appropriate for this purpose.

In general, the content of sodium starch glycolate is not limited in any way as long as it ensures good dissolution properties of known compound A. For example, it is 5% to 90% by weight, or alternatively, 5% to 70% by weight, based on the pharmaceutical composition of the present invention. Likewise, it is 10% to 1500% by weight, or alternatively, 10% to 1100% by weight, relative to the amount of the cocrystal of known compound A and L-proline.

The solid pharmaceutical composition of the present invention may optionally be further supplemented, as appropriate, with various pharmaceutical additives before being formulated. Such pharmaceutical additives are not limited in any way as long as they are pharmaceutically acceptable and pharmacologically acceptable. Examples include excipients, binders, disintegrants, acidulants, blowing agents, artificial sweeteners, flavorings, lubricants, coloring agents, stabilizers, buffering agents, antioxidants, surfactants, coating agents and the like.

Excipients include D-mannitol, lactose and the like.

Binders include, for example, hydroxypropylmethylcellulose, hydroxypropylcellulose, gum arabic and the like.

Acidulants include, for example, citric acid, tartaric acid, malic acid and the like.

Blowing agents include, for example, sodium hydrogen carbonate and the like.

Artificial sweeteners include, for example, saccharin sodium, dipotassium glycyrrhizinate, aspartame, stevia, thaumatin and the like.

Flavorings include, for example, lemon, lemon lime, orange, menthol and the like.

Lubricants include, for example, magnesium stearate, calcium stearate, sucrose fatty acid ester, polyethylene glycol, talc, stearic acid and the like.

Coloring agents include, for example, yellow iron sesquioxide, red iron sesquioxide, food yellow Nos. 4 and 5, food red Nos. 3 and 102, food blue No. 3 and the like.

Buffering agents include citric acid, succinic acid, fumaric acid, tartaric acid, ascorbic acid or salts thereof, glutamic acid, glutamine, glycine, aspartic acid, alanine, arginine or salts thereof, magnesium oxide, zinc oxide, magnesium hydroxide, phosphoric acid, boric acid or salts thereof and the like.

Antioxidants include, for example, ascorbic acid, dibutylhydroxytoluene, propyl gallate and the like.

Surfactants include, for example, Polysorbate 80, sodium lauryl sulfate, polyoxyethylene hydrogenated castor oil and the like.

Coating agents include talc, polyethylene glycol, hypromellose, titanium dioxide and the like.

These pharmaceutical additives may be added alone or in combination as appropriate in suitable amounts.

The content of pharmaceutical additive(s) is 0.1% to 70% by weight, based on the pharmaceutical composition of the present invention.

The pharmaceutical composition of the present invention can be formulated into various dosage forms, including tablets, capsules, powders, granules, dry syrups and the like. In a certain embodiment, the solid pharmaceutical composition of the present invention is in tablet form.

Formulations of various dosage forms may be produced in any known manner.

The pharmaceutical composition of the present invention can be produced, for example, by any known process including the steps of grinding, wet granulation, drying, tableting, film coating and the like.

For example, the solid pharmaceutical composition of the present invention in the form of powders, granules or dry syrups can be produced by a process including the steps of (1) mixing a cocrystal of known compound A and L-proline with crystalline cellulose, and (2) granulating the resulting mixture by wet granulation. In a case where the above various pharmaceutical additives are used as needed, these pharmaceutical additives may be added at any stage, e.g., during step (1), between steps (1) and (2), or during step (2). In a certain embodiment, a disintegrant is used as a pharmaceutical additive, and sodium starch glycolate and L-HPC are presented as examples of the disintegrant.

For example, the solid pharmaceutical composition of the present invention in the form of tablets can be produced by a process including the steps of (1) mixing a cocrystal of known compound A and L-proline with crystalline cellulose, (2) granulating the resulting mixture by wet granulation, and (3) subjecting the granulated product to compression molding. In a case where the above various pharmaceutical additives are used as needed, these pharmaceutical additives may be added at any stage, e.g., during step (1), between steps (1) and (2), during step (2), or between steps (2) and (3). In a certain embodiment, a disintegrant is used as a pharmaceutical additive, and sodium starch glycolate and L-HPC are presented as examples of the disintegrant.

The cocrystal of known compound A and L-proline, crystalline cellulose, and the pharmaceutical additive(s) may each be adjusted to any suitable size by being subjected to a grinding step prior to the mixing step. In the grinding step, any apparatus or means may be used as long as it generally allows pharmaceutical grinding of the drug and/or the pharmaceutical additive(s). In the mixing step of the individual components, which is subsequent to grinding, any apparatus or means may be used as long as it generally allows pharmaceutical mixing of the individual components into a uniform state.

A granulator is used for wet granulation of the mixture to prepare a granulated product. Examples of a granulator include a fluidized bed granulator, a tumbling fluidized bed granulator, a stirring granulator and the like.

In wet granulation, a binder is used. The binder may be added as an aqueous solution to a mixture containing a cocrystal of known compound A and L-proline as well as crystalline cellulose. Although the speed of binder addition will vary depending on the type of granulation method or the scale of production, for example, the binder solution may be added at a speed of 1 to 30 g/minute, or alternatively, 5 to 20 g/minute in the case of 1 kg scale production by fluidized bed granulation. Wet granulation may be accomplished, for example, in a temperature range of 15°C to 35°C. During wet granulation, in addition to the aqueous binder solution, another water-containing component may be added. In one embodiment, a binder may be added to a mixture containing a cocrystal of known compound A and L-proline as well as crystalline cellulose, followed by granulation while adding water. The water may be added such that the maximum moisture content during granulation is 5% to 40% by weight or 5% to 30% by weight. The maximum moisture content during granulation refers to a maximum moisture content measured for a mixture during granulation, mainly refers to a moisture content in a mixture at a time point when addition of the aqueous binder solution (or water) is completed. The moisture content in such a mixture may be measured with a halogen moisture analyzer (Mettler-Toledo Inc.) by allowing the mixture to stand at 105°C for 5 minutes.

The solid pharmaceutical composition of the present invention comprises a cocrystal structure between known compound A and L-proline, and the cocrystal structure should be maintained even during preparation of the composition (i.e., L-proline should not be released from the cocrystal to prevent known compound A from being left in free form). To maintain the cocrystal structure, production conditions are preferably controlled such that a strong shearing force and excess moisture are not applied to a mixture to be granulated. Since a strong shearing force is excluded, the pharmaceutical composition of the present invention is preferably granulated by use of fluidized bed granulation.

The granulated product thus prepared may be dried using any means. For example, it is possible to use a fluidized bed granulator, a Multiplex, a shelf dryer, or other dryers. The drying temperature is set to, for example, 40°C to 90°C.

The granulated product is then tableted to produce tablets. Any tableting technique may be used for this purpose as long as it generally allows pharmaceutical production of compression molded products. Examples include techniques in which a granulated product is tableted in admixture with a disintegrant, a lubricant and the like. Any type of tablet machine may be used for this purpose as long as it generally allows pharmaceutical production of compression molded products. Examples include a rotary tablet machine, a single-shot tablet machine and the like. The tablet hardness is set to, for example, 40 to 250 N, or alternatively, 50 to 200 N.

After tableting, the tablet surface may be coated with a film coating. Any technique may be used for this purpose as long as it generally allows pharmaceutical tablet coating. Examples include pan coating processes and the like. Any type of film coating agent may be used for this purpose as long as it is generally used as a pharmaceutical additive for pharmaceutical tablet coating. Film coating agents may be added alone or in combination as appropriate in suitable amounts.

In general, the coating rate is not limited in any way as long as the tablet surface can be coated. For example, it is 1.0% by weight or more to 5.0% by weight or less, relative to uncoated tablets before coating.

Any method may be used to produce the pharmaceutical composition of the present invention or a pharmaceutical formulation thereof, as long as it allows production of pharmaceutical formulations having the desired effects of the present invention by the method described above or an appropriate combination of methods known per se.

Use of crystalline cellulose in the present invention is intended for the manufacture of a solid pharmaceutical composition that maintains good dissolution properties and dissolution stability of known compound A.

### EXAMPLE

The present invention will be further described in more detail below by way of the following examples, comparative example and test examples, which are not intended to limit the scope of the invention. A cocrystal of known compound A and L-proline (1:1) was prepared as described in WO2007/114475 and used. The compositions of the examples and the comparative example were prepared based on the formulations shown in Tables 3-5, 7, and 8. It should be noted that each numeral in each table indicates the weight (g) of each component used.

### <Example 1>

As an excipient, crystalline cellulose was used to prepare tablets based on the formulation shown in Table 3.

After the cocrystal of known compound A and L-proline was mixed with crystalline cellulose (product name: Ceolus PH101, Asahi Kasei Corporation, Japan, the same applies hereinafter), an aqueous solution (10% by weight) of hydroxypropylcellulose (product name: HPC-L, Nippon Soda Co., Ltd., Japan, the same applies hereinafter) was sprayed as a binder solution (addition speed: 10 g/minute) to effect fluidized bed granulation (granulator (product name: tumbling fluidized bed granulating, drying and coating apparatus, Powrex Corp., Japan), granulation temperature: 24°C to 25°C, granulation time: 48 minutes). The mixture during granulation had a maximum moisture content value of 29.2%, and the resulting granulated product had an average particle size of 292 µm. The resulting granulated product was dried (75°C, 40 minutes) and then mixed with sodium starch glycolate (product name: Primojel, DMV, the same applies hereinafter) and magnesium stearate (product name: Parteck LUB MST, Merck & Co., Inc., the same applies hereinafter), followed by tableting to obtain tablets (A-1) (punch diameter: 9.5 mm x 11.4R, tableting pressure: 6 kN). The moisture content value during granulation was measured as follows: the mixture was sampled from the granulator at an interval of 12 minutes from the start until the completion of spraying the binder solution, and allowed to stand at 105°C for 5 minutes for measurement with a halogen moisture analyzer (Mettler-Toledo Inc.). The maximum moisture content value was measured after the completion of spraying.

Except that croscarmellose sodium (product name: Ac-Di-Sol, FMC BioPolymer, the same applies hereinafter), low-substituted hydroxypropylcellulose whose hydroxypropoxyl group content is 10% to 12.9% (product name: L-HPC, Shin-Etsu Chemical Co., Ltd., Japan, the same applies hereinafter) or crospovidone (product name: Kollidon CL, BASF, the same applies hereinafter) was used instead of sodium starch glycolate, the same procedure as shown above was repeated to obtain tablets A-2 to A-4 (A-2: maximum moisture content value: 29.2%, punch diameter: 9.5 mm x 11.4R, tableting pressure: 5 kN; A-3: maximum moisture content value: 29.2%, punch diameter: 9.5 mm x 11.4R, tableting pressure: 5 kN; A-4: maximum moisture content value: 29.2%, punch diameter: 9.5 mm x 11.4R, tableting pressure: 5 kN).

### <Comparative Example 1>

Except that D-mannitol (product name: PEARITOL 50C, Roquette, the same applies hereinafter) was used as an excipient instead of crystalline cellulose, the same procedure as shown in Example 1 was repeated to obtain a granulated product (average particle size: 144 µm) and tablets B-1 to B-4 (B-1: maximum moisture content value: 3.7%, punch diameter: 9.5 mm x 11.4R, tableting pressure: 10 kN; B-2: maximum moisture content value: 3.7%, punch diameter: 9.5 mm x 11.4R, tableting pressure: 13 kN; B-3: maximum moisture content value: 3.7%, punch diameter: 9.5 mm x 11.4R, tableting pressure: 11 kN; B-4: maximum moisture content value: 3.7%, punch diameter: 9.5 mm x 11.4R, tableting pressure: 11 kN).

### <Comparative Example 2>

Except that anhydrous dibasic calcium phosphate (product name: GS CALICA, Kyowa Chemical Industry Co., Ltd) was used as an excipient instead of crystalline cellulose, the same procedure as shown in Example 1 was repeated to obtain a granulated product and tablets C-1 to C-4 (C-1: maximum moisture content value: 8.1%, punch diameter: 9.5 mm x 11.4R, tableting pressure: 15 kN; C-2: maximum moisture content value: 8.1%, punch diameter: 9.5 mm x 11.4R, tableting pressure: 15 kN; C-3: maximum moisture content value: 8.1%, punch diameter: 9.5 mm x 11.4R, tableting pressure: 9 kN; C-4: maximum moisture content value: 8.1%, punch diameter: 9.5 mm x 11.4R, tableting pressure: 13 kN).

[Table 3]

**Table 3**

| | A-1 | A-2 | A-3 | A-4 |
|---|---|---|---|---|
| Cocrystal of known compound A and L-proline | 128.6 | 128.6 | 128.6 | 128.6 |
| Crystalline cellulose | 404.2 | 404.2 | 404.2 | 404.2 |
| Sodium starch glycolate, | 144,0 | - | - | - |
| Croscarmellose sodium | - | 144.0 | - | - |
| Low-substituted hydroxypropylcellulose | - | - | 144.0 | - |
| Crospovidone | - | - | - | 144.0 |
| Hydroxypropylcellulose | 36.0 | 36.0 | 36.0 | 36.0 |
| Magnesium stearate | 7.2 | 7.2 | 7.2 | 7.2 |
| Total | 720.0 | 720.0 | 720.0 | 720.0 |

[Table 4]

**Table 4**

| | B-1 | B-2 | B-3 | B-4 |
|---|---|---|---|---|
| Cocrystal of known compound A and L-proline | 128.6 | 128.6 | 128.6 | 128.6 |
| D-mannitol | 404.2 | 404.2 | 404.2 | 404.2 |
| Sodium starch glycolate | 144.0 | - | - | - |
| Croscarmellose sodium | - | 144.0 | - | - |
| Low-substituted hydroxypropylcellulose | - | - | 144.0 | - |
| Crospovidone | - | - | - | 144.0 |
| Hydroxypropylcellulose | 36.0 | 36.0 | 36.0 | 36.0 |
| Magnesium stearate | 7.2 | 7.2 | 7.2 | 7.2 |
| Total | 720.0 | 720.0 | 720.0 | 720.0 |

[Table 5]

**Table 5**

| | C-1 | C-2 | C-3 | C-4 |
|---|---|---|---|---|
| Cocrystal of known compound A and L-proline | 128.6 | 128.6 | 128.6 | 128.6 |
| Anhydrous dibasic calcium phosphate | 404.2 | 404.2 | 404.2 | 404.2 |
| Sodium starch glycolate | 144.0 | - | - | - |
| Croscarmellose sodium | - | 144.0 | - | - |
| Low-substituted hydroxypropylcellulose | - | - | 144.0 | - |
| Crospovidone | - | - | - | 144.0 |
| Hydroxypropylcellulose | 36.0 | 36.0 | 36.0 | 36.0 |
| Magnesium stearate | 7.2 | 7.2 | 7.2 | 7.2 |
| Total | 720.0 | 720.0 | 720.0 | 720.0 |

### < Test Example 1>

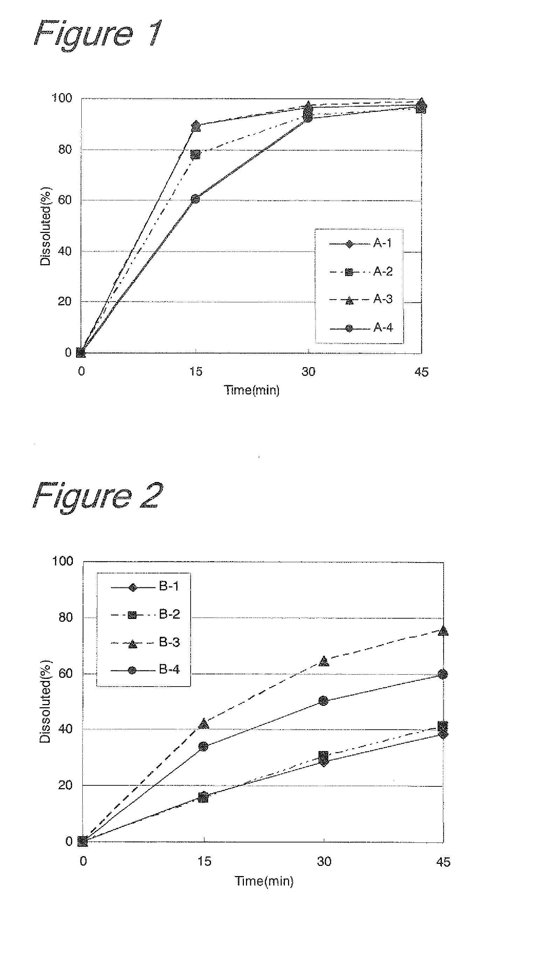

The individual tablets obtained in Example 1, Comparative Example 1, and Comparative Example 2 were subjected to a dissolution test to study their dissolution immediately after formulation (at the start of storage). The dissolution test was accomplished by the paddle method described in the 15th revised Japanese Pharmacopoeia. The test solution used was 900 mL of dissolution test solution 1 (0.1 N aqueous hydrochloric acid). The rotation speed of paddles was set to 50 rotations/minute. The dissolution rate of known compound A at 30 minutes after the start of the test is shown in Table 6. Moreover, the dissolution profile is shown in Figures 1-3.

As can be seen from Table 6 and Figures 1-3, the tablets prepared using crystalline cellulose showed good dissolution properties as compared to the tablets prepared using D-mannitol or anhydrous dibasic calcium phosphate. Moreover, the dissolution properties of the tablets prepared using crystalline cellulose were found to be good, regardless of the type of disintegrant.

This suggests that when the cocrystal of known compound A and L-proline is combined with crystalline cellulose, it is possible to provide a solid pharmaceutical composition having good dissolution properties.

[Table 6]

**Table 6**

| Tablet | Dissolution rate (%) |
|---|---|
| A-1 | 97 |
| A-2 | 94 |
| A-3 | 97 |
| A-4 | 92 |
| B-1 | 29 |
| B-2 | 31 |
| B-3 | 65 |
| B-4 | 50 |
| C-1 | 70 |
| C-2 | 69 |
| C-3 | 72 |
| C-4 | 67 |

### <Example 2>

In the same manner as shown in Example 1, after the cocrystal of known compound A and L-proline was mixed with crystalline cellulose, the aqueous hydroxypropylcellulose solution was sprayed as a binder solution to effect fluidized bed granulation (maximum moisture content value during granulation: 29.2%, average particle size: 144 µm). The resulting granulated product was dried and then mixed with sodium starch glycolate and magnesium stearate, followed by tableting to obtain the solid pharmaceutical composition of the present invention (punch diameter: 9.5 mm x 11.4R, tableting pressure: 7 kN).

### <Example 3>

In the same manner as shown in Example 1, after the cocrystal of known compound A and L-proline was mixed with D-mannitol, crystalline cellulose and sodium starch glycolate, the aqueous hydroxypropylcellulose solution was sprayed as a binder solution to effect fluidized bed granulation (maximum moisture content value during granulation: 15.4%, average particle size: 248 µm). The resulting granulated product was dried and then mixed with magnesium stearate, followed by tableting to obtain the solid pharmaceutical composition of the present invention (punch diameter: 9.5 mm x 11.4R, tableting pressure: 7 kN).

### <Example 4>

In the same manner as shown in Example 1, after the cocrystal of known compound A and L-proline was mixed with D-mannitol, crystalline cellulose and sodium starch glycolate, the aqueous hydroxypropylcellulose solution was sprayed as a binder solution to effect fluidized bed granulation (maximum moisture content value during granulation: 20.1%, average particle size: 164 µm). The resulting granulated product was dried and then mixed with magnesium stearate, followed by tableting to obtain the solid pharmaceutical composition of the present invention (punch diameter: 10.0 mm x 15.0R, tableting pressure: 8 kN).

### <Example 5>

In the same manner as shown in Example 1, after the cocrystal of known compound A and L-proline was mixed with D-mannitol, crystalline cellulose and sodium starch glycolate, the aqueous hydroxypropylcellulose solution was sprayed as a binder solution to effect fluidized bed granulation (maximum moisture content value during granulation: 23.2%, average particle size: 115 µm). The resulting granulated product was dried and then mixed with magnesium stearate, followed by tableting to obtain the solid pharmaceutical composition of the present invention (punch diameter: 9.5 mm x 11.4R, tableting pressure: 8 kN).

### <Example 6>

In the same manner as shown in Example 1, after the cocrystal of known compound A and L-proline was mixed with crystalline cellulose, the aqueous hydroxypropylcellulose solution was sprayed as a binder solution to effect fluidized bed granulation (maximum moisture content value during granulation: 29.2%, average particle size: 292 µm). The resulting granulated product was dried and then mixed with sodium starch glycolate and magnesium stearate, followed by tableting to obtain the solid pharmaceutical composition of the present invention (punch diameter: 9.5 mm x 11.4R, tableting pressure: 6 kN).

### <Example 7>

In the same manner as shown in Example 1, after the cocrystal of known compound A and L-proline was mixed with crystalline cellulose, the aqueous hydroxypropylcellulose solution was sprayed as a binder solution to effect fluidized bed granulation (maximum moisture content value during granulation: 29.2%, average particle size: 292 µm). The resulting granulated product was dried and then mixed with magnesium stearate, followed by tableting to obtain the solid pharmaceutical composition of the present invention (punch diameter: 9.5 mm x 11.4R, tableting pressure: 5 kN).

### <Example 8>

In the same manner as shown in Example 1, after the cocrystal of known compound A and L-proline was mixed with D-mannitol, crystalline cellulose and low-substituted hydroxypropylcellulose, the aqueous hydroxypropylcellulose solution was sprayed as a binder solution to effect fluidized bed granulation (maximum moisture content value during granulation: 10.4%, average particle size: 231 µm). The resulting granulated product was dried and then mixed with magnesium stearate, followed by tableting to obtain the solid pharmaceutical composition of the present invention (punch diameter: 9.5 mm x 11.4R, tableting pressure: 5 kN).

### <Example 9>

In the same manner as shown in Example 1, after the cocrystal of known compound A and L-proline was mixed with D-mannitol and crystalline cellulose, the aqueous hydroxypropylcellulose solution was sprayed as a binder solution to effect fluidized bed granulation (maximum moisture content value during granulation: 16.6%, average particle size: 197 µm). The resulting granulated product was dried and then mixed with low-substituted hydroxypropylcellulose and magnesium stearate, followed by tableting to obtain the solid pharmaceutical composition of the present invention (punch diameter: 9.5 mm x 11.4R, tableting pressure: 5 kN).

### <Example 10>

In the same manner as shown in Example 1, after the cocrystal of known compound A and L-proline was mixed with D-mannitol and crystalline cellulose, the aqueous hydroxypropylcellulose solution was sprayed as a binder solution to effect fluidized bed granulation (maximum moisture content value during granulation: 16.6%, average particle size: 197 µm). The resulting granulated product was dried and then mixed with low-substituted hydroxypropylcellulose and magnesium stearate, followed by tableting to obtain the solid pharmaceutical composition of the present invention (punch diameter: 9.5 mm x 11.4R, tableting pressure: 5 kN).

[Table 7]

**Table 7**

| | Example 2 | Example 3 | Example 4 | Example 5 | Example 6 |
|---|---|---|---|---|---|
| Cocrystal of known compound A and L-proline | 128.6 | 128.6 | 128.6 | 128.6 | 128.6 |
| D-mannitol | - | 113.6 | 125.6 | 125.6 | - |
| Crystalline cellulose | 404.2 | 56.8 | 278.6 | 293.0 | 404.2 |
| Sodium starch glycolate | 36.0 | 377.8 | 144.0 | 144.0 | 144.0 |
| Hydroxypropylcellulose | 36.0 | 36.0 | 36.0 | 21.6 | 36.0 |
| Magnesium stearate | 7.2 | 7.2 | 7.2 | 7.2 | 7.2 |
| Total | 612.0 | 720.0 | 720.0 | 720.0 | 720.0 |

[Table 8]

**Table 8**

| | Example 7 | Example 8 | Example 9 | Example 10 |
|---|---|---|---|---|
| Cocrystal of known compound A and L-proline | 128.6 | 128.6 | 128.6 | 128.6 |
| D-mannitol | - | 125.6 | 125.6 | 125.6 |
| Crystalline cellulose | 404.2 | 278.6 | 278.6 | 278.6 |
| Low-substituted hydroxypropylcellulose | - | 144.0 | 144.0 | 36.0 |
| Hydroxypropylcellulose | 36.0 | 36.0 | 36.0 | 36.0 |
| Magnesium stearate | 7.2 | 7.2 | 7.2 | 7.2 |
| Total | 576.0 | 720.0 | 720.0 | 612.0 |

### <Test Example 2>: Crystalline analysis

The compositions obtained in Examples 2 to 10 were evaluated by powder X-ray diffractometry for their crystal structure immediately after formulation. As a result, peaks corresponding to the cocrystal of known compound A and L-proline were observed, whereas there was no peak corresponding to free form of known compound A. This indicated that the cocrystal structure between known compound A and L-proline was maintained even after formulation (Table 9).

### <Test Example 3>: Evaluation of dissolution properties and dissolution stability

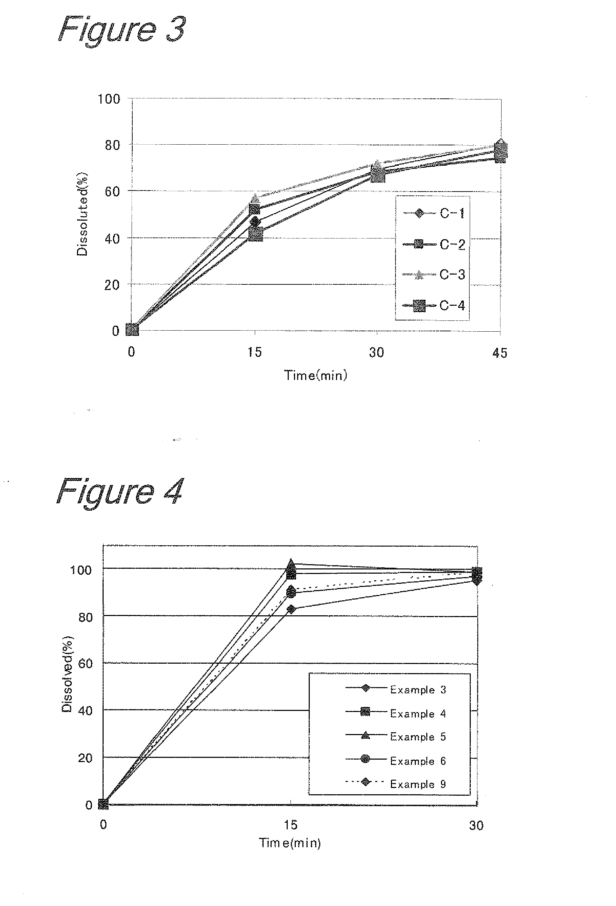

The compositions obtained in Examples 2 to 10 were subjected to a dissolution test to study their dissolution immediately after formulation (at the start of storage) and after storage at 40°C and 75% relative humidity for 6 months. The dissolution test was accomplished by the paddle method described in the 15th revised Japanese Pharmacopoeia. The test solution used was 900 mL of dissolution test solution 1 (0.1 N aqueous hydrochloric acid). The rotation speed of paddles was set to 50 rotations/minute. The dissolution rate of known compound A at 30 minutes after the start of the test is shown in Table 9. Moreover, the dissolution profile is shown in Figure 4.

[Table 9]

**Table 9**

| | Crystal structure | Dissolution rate at the start of storage 30 minute value | Dissolution rate after storage 30 minute value |
|---|---|---|---|
| Example 2 | Cocrystal | 85% | - |
| Example 3 | Cocrystal | 95% | - |
| Example 4 | Cocrystal | 99% | 97% |
| Example 5 | Cocrystal | 99% | 96% |
| Example 6 | Cocrystal | 97% | - |
| Example 7 | Cocrystal | 83% | - |
| Example 8 | Cocrystal | 83% | - |
| Example 9 | Cocrystal | 99% | - |
| Example 10 | Cocrystal | 73% | - |

As shown in Examples 2 to 10, the dissolution rate after 30 minutes reached a high value in each case. When crystalline cellulose was included in the formulation and wet granulation was used, significant improvements were observed in dissolution properties.

Test Examples 1 and 2 demonstrated that the solid pharmaceutical compositions containing the cocrystal of known compound A and L-proline in combination with crystalline cellulose maintained the cocrystal structure between known compound A and L-proline and also showed good dissolution properties. It is suggested that inclusion of crystalline cellulose in the formulation allows a reduction in the strong aggregation tendency, which is characteristic of the cocrystal of compound A and L-proline, and also allows an improvement in dispersibility.

### INDUSTRIAL APPLICABILITY

The present invention relates to a solid pharmaceutical composition, which comprises a cocrystal of known compound A and L-proline and which maintains good dissolution properties and dissolution stability of known compound A, a method for producing the same, and the use of crystalline cellulose for the manufacture of the solid pharmaceutical composition. The present invention ensures improved bioavailability (BA) and a pharmacologically sufficient therapeutic effect, due to good dissolution properties of the cocrystal of known compound A and L-proline.

## Claims

1. A solid pharmaceutical composition comprising a cocrystal of (1S)-1,5-anhydro-1-[3-(1-benzothien-2-ylmethyl)-4-fluorophenyl]-D-glucitol and L-proline, in combination with crystalline cellulose.

2. The solid pharmaceutical composition according to claim 1, wherein the content of crystalline cellulose in the pharmaceutical composition is 5% by weight or more to 90% by weight or less.

3. The solid pharmaceutical composition according to claim 1 or 2, which further comprises a disintegrant.

4. The solid pharmaceutical composition according to claim 3, wherein the disintegrant is one or more members selected from the group consisting of sodium starch glycolate, and hydroxypropylcellulose whose hydroxypropoxyl group content is 5% by weight or more to less than 16% by weight.

5. The solid pharmaceutical composition according to claim 3 or 4, wherein the content of the disintegrant in the pharmaceutical composition is 5% by weight or more to 90% by weight or less.

6. The solid pharmaceutical composition according to any one of claims 1 to 5, wherein 65% or more of (1S)-1,5-anhydro-1-[3-(1-benzothien-2-ylmethyl)-4-fluorophenyl]-D-glucitol is dissolved in 30 minutes, as analyzed by the dissolution test described in the 15th revised Japanese Pharmacopoeia.

7. A method for producing a solid pharmaceutical composition, which comprises the steps of:
(1) mixing a cocrystal of (1S)-1,5-anhydro-1-[3-(-benzothien-2-ylmethyl)-4-fluorophenyl]-D-glucitol and L-proline with crystalline cellulose; and
(2) granulating the resulting mixture by wet granulation while maintaining the cocrystal structure between (1S)-1,5-anhydro-1-[3-(1-benzothien-2-ylmethyl)-4-fluorophenyl]-D-glucitol and L-proline.

8. The method according to claim 7, which further comprises the step of (3) subjecting the granulated product to compression molding.

9. The method according to claim 7 or 8, wherein the content of crystalline cellulose in the pharmaceutical composition is 5% by weight or more to 90% by weight or less.

10. The method according to claim 7, which further comprises adding a disintegrant during step (1), between steps (1) and (2), during step (2), or after step (2).

11. The method according to claim 10, wherein the disintegrant is one or more members selected from the group consisting of sodium starch glycolate, and hydroxypropylcellulose whose hydroxypropoxyl group content is 5% by weight or more to less than 16% by weight.

12. The method according to claim 10 or 11, wherein the content of the disintegrant in the pharmaceutical composition is 5% by weight or more to 90% by weight or less.

13. The method according to any one of claims 7 to 12, which is intended to produce a solid pharmaceutical composition that allows 65% or more of (1S)-1,5-anhydro-1-[3-(1-benzothien-2-ylmethyl)-4-fluorophenyl]-D-glucitol to be dissolved in 30 minutes, as analyzed by the dissolution test described in the 15th revised Japanese Pharmacopoeia.

14. The method according to claim 7, wherein the wet granulation is performed such that the solid pharmaceutical composition has a maximum moisture content value of 5% to 30% by weight during granulation.

15. Use of crystalline cellulose for the manufacture of a solid pharmaceutical composition comprising a cocrystal of (1S)-1,5-anhydro-1-[3-(1-benzothien-2-ylmethyl)-4-fluorophenyl]-D-glucitol and L-proline.
